# EUROPEAN PATENT SPECIFICATION

(11) **EP 3 876 241 B1**
(45) Date of publication and mention of the grant of the patent: **10.09.2025**
(21) Application number: 19880330.6
(22) Date of filing: 24.10.2019
(51) Int. Cl.: G16C 20/30, G16C 20/90, G16C 60/00, G16Z 99/00, C22C 1/02, G06N 3/04, G06N 3/08, G06N 20/00, G16C 20/70

(54) **THERMODYNAMIC EQUILIBRIUM STATE PREDICTION DEVICE, PREDICTION METHOD AND PREDICTION PROGRAM**
VORRICHTUNG, VERFAHREN UND PROGRAMM ZUR VORHERSAGE DES THERMODYNAMISCHEN GLEICHGEWICHTSZUSTANDS
DISPOSITIF DE PRÉDICTION D'ÉTAT D'ÉQUILIBRE THERMODYNAMIQUE, PROCÉDÉ DE PRÉDICTION ET PROGRAMME DE PRÉDICTION

(30) Priority: 31.10.2018 JP 2018206017
(43) Date of publication of application: 08.09.2021
(73) Proprietor: Resonac Corporation, Tokyo 105-7325 (JP)
(72) Inventor: OKUNO, Katsuki, Tokyo 105-8518 (JP); OKUNO, Yoshishige, Tokyo 105-8518 (JP)
(74) Representative: Mewburn Ellis LLP
(86) International application number: PCT/JP2019/041716
(87) International publication number: WO 2020/090617

(56) References cited:
- CN-A- 102 663 498
- JP-A- 2018 010 428
- JP-A- 2018 158 830
- JP-A- H1 055 348
- US-A1- 2014 236 548
- US-A1- 2015 106 035
- US-A1- 2016 034 614
- ISLAM N. ET AL: "Machine learning for phase selection in multi-principal element alloys", COMPUTATIONAL MATERIALS SCIENCE, ELSEVIER, AMSTERDAM, NL, vol. 150, 20 April 2018 (2018-04-20), pages 230 - 235, XP085399871, ISSN: 0927-0256, DOI: 10.1016/J.COMMATSCI.2018.04.003
- MOHAMMADI A. H. ET AL: "Determining phase diagrams of tetrahydrofuran+methane, carbon dioxide or nitrogen clathrate hydrates using an artificial neural network algorithm", CHEMICAL ENGINEERING SCIENCE, OXFORD, GB, vol. 65, no. 22, 15 November 2010 (2010-11-15), pages 6059 - 6063, XP027394908, ISSN: 0009-2509, [retrieved on 20100727]
- GOLDSMITH B. R. ET AL: "Machine learning for heterogeneous catalyst design and discovery", AICHE JOURNAL, vol. 64, no. 7, 25 May 2018 (2018-05-25), US, pages 2311 - 2323, XP055769277, ISSN: 0001-1541, DOI: 10.1002/aic.16198
- MINAMOTO, SATOSHI ET AL.: "Thermodynamic analysis by using Materials Integration", 2017 AUTUMN LECTURE CONFERENCE OF THE JAPAN INSTITUTE OF METALS, SEPTEMBER 6-8, 2017, vol. 161, 23 August 2017 (2017-08-23), pages 1, XP009527204

## Description

### Technical Field

The present invention relates to a prediction device, a prediction method, and a prediction program for predicting a thermodynamic equilibrium state.

### Background Art

A calculation of phase diagram and thermodynamics (CALPHAD) method is known as a computer simulation method of predicting thermodynamic equilibrium states of alloys, ceramics, aqueous solutions, chemical reactions, and the like (e. g., Patent Document 1).

Islam N. et al. (2018-04-20) "Machine learning for phase selection in multi-principal element alloys", COMPUTATIONAL MATERIALS SCIENCE, vol. 150, pages 230-235, discloses machine learning for phase selection in multi-principal element alloys.

US2016/034614A1 discloses a device and article of manufacture to predict material properties of a cast aluminum-based component. In one form, a computer-based system includes numerous computation modules programmably cooperative with one another such that upon receipt of data that corresponds to the cast aluminum-based component, the modules provide performance indicia of the material. The modules include a thermodynamic calculation module, a thermal-physical property module, a mechanical property module and a materials selection or alloy design module. The combination of the modules along with known material and geometric databases-in addition to microstructural and defect databases-promotes the generation of materials properties needed for casting design, casting process simulation, CAE nodal property mapping and durability analysis.

Mohammadi A. H. et al. (2010) "Determining phase diagrams of tetrahydrofuran+methane, carbon dioxide or nitrogen clathrate hydrates using an artificial neural network algorithm", CHEMICAL ENGINEERING SCIENCE, vol. 65, no. 22, pages 6059-6063, discloses determining phase diagrams of tetrahydrofuran+methane, carbon dioxide or nitrogen clathrate hydrates using an artificial neural network algorithm.

### Related Art Document

### Patent Document

[Patent Document 1] Japanese Laid-open Patent Application Publication No. 2014-48208

### Summary of the Invention

The invention is defined in the appended claims, in particular, independent claims.

### Summary of Related Disclosure

### Problem to be Solved by the Invention

The CALPHAD method is a simulation method that searches for a state of providing minimal Gibbs energy as an equilibrium state, and takes a long time for the calculation. To perform a thermodynamic equilibrium calculation for a single material composition to predict a phase diagram, the CALPHAD method can be performed within a realistic time, but it is too time consuming and impractical to predict phase diagrams and perform screening for a large number of material compositions. For an alloy material, for example, it would take about 250 hours in total to calculate phase diagrams of all alloy compositions to screen 10,000 explanatory variables (i.e., sets of compositions and manufacturing conditions) because it takes about 90 seconds to calculate for each set.

It is an object of the present invention to provide a prediction device, a prediction method, and a prediction program for predicting the thermodynamic equilibrium state in a short time.

### Means for Solving the Problem

The present invention has the following configuration.
[1] According to a first aspect of the present invention there is provided a prediction device as specified in claim 1.
[2] The prediction device as described in [1], wherein the model is a multi-layer neural network and the model training unit trains the model by using deep learning.
[3] The prediction device as described in [2], wherein the target variables that are output from the model are phase fractions of the target material in the thermodynamic equilibrium state, and wherein a softmax function is used for neurons in an output layer of the multi-layer neural network.
[4] The prediction device as described in any one of [1] to [3], wherein the training data generating unit generates explanatory variables including a combination of predetermined ranges of the design conditions, calculates the target variables by using a CALPHAD method, and generates the training data including the generated explanatory variables and the calculated target variables.
[5] The prediction device as described in any one of [1] to [4], wherein the target material is an aluminum alloy, the explanatory variables include a composition and a manufacturing condition of the aluminum alloy, and the target variables include phase fractions of the aluminum alloy in the thermodynamic equilibrium state.
[6] According to a second aspect of the present invention there is provided a prediction method as specified in claim 6.
[7] According to a third aspect of the present invention there is provided a prediction program as specified in claim 7.

### Effect of the Invention

According to the present invention, a prediction device, a prediction method, and a prediction program, for predicting the thermodynamic equilibrium, that calculate the thermodynamic equilibrium state in a short time can be provided.

### Brief Description of the Drawings

FIG. 1 is a block diagram illustrating a schematic configuration of a prediction device according to an embodiment;
FIG. 2 is a drawing illustrating an example of training data of a four-component system;
FIG. 3 is a drawing illustrating a trained result obtained when the training data of the four-component system illustrated in FIG. 2 is used;
FIG. 4 is a drawing illustrating an example of training data of a nine-component system;
FIG. 5 is a drawing illustrating a trained result obtained when the training data of the nine-component system illustrated in FIG. 4 is used;
FIG. 6 is a block diagram illustrating a hardware configuration of the prediction device; and
FIG. 7 is a flowchart of a process of predicting a thermodynamic equilibrium state, performed by a prediction device 1 according to the embodiment.

### Embodiment for Carrying Out the Invention

In the following, an embodiment will be described with reference to the accompanying drawings. In order to facilitate the understanding of the description, the same elements in each drawing are referenced by the same reference signs to the extent possible, and the overlapping description is omitted.

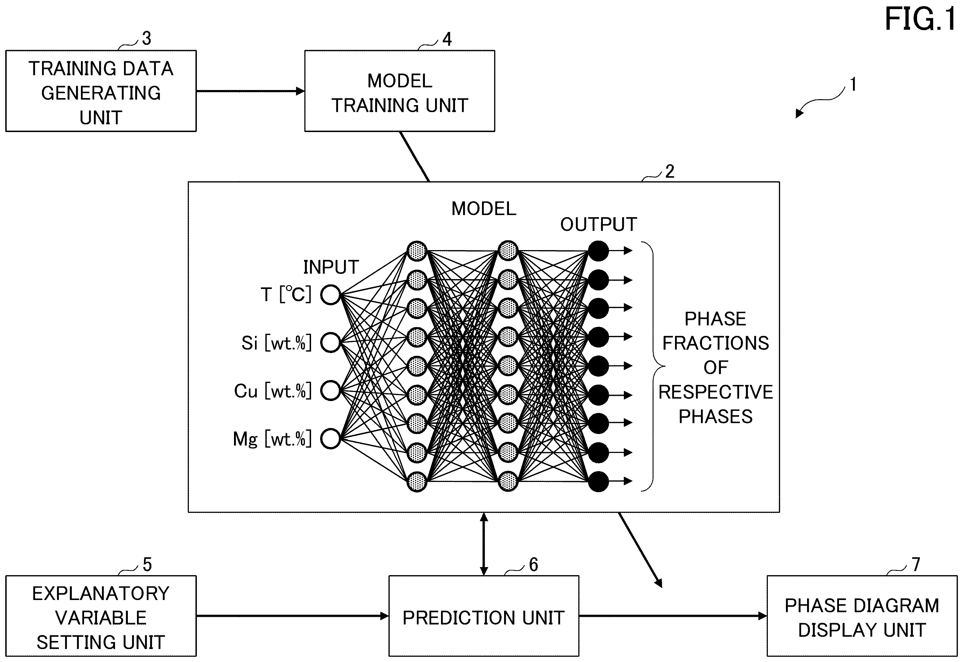

Referring to FIGs. 1 to 5, a configuration of a prediction device 1 for predicting a thermodynamic equilibrium state (which will be hereinafter simply referred to as "the prediction device 1") according to the embodiment will be described. FIG. 1 is a block diagram illustrating a schematic configuration of the prediction device 1 according to the embodiment. The prediction device 1 is a device for predicting the thermodynamic equilibrium state or a phase diagram of a target material containing a material that consists of multiple compositions or a material produced through a combination of multiple manufacturing conditions. In the present embodiment, an aluminum alloy is used as an example of the target material to be predicted.

As illustrated in FIG. 1, the prediction device 1 includes a model 2, a training data generating unit 3, a model training unit 4, an explanatory variable setting unit 5, a prediction unit 6, and a phase diagram display unit 7.

The model 2 outputs target variables related to the thermodynamic equilibrium state (i.e., phase fractions of compounds in the thermodynamic equilibrium state) based on input explanatory variables related to design conditions of the target material (a composition and a manufacturing condition of the aluminum alloy). The model 2 is a supervised learning model, and as a preliminary step for predicting the thermodynamic equilibrium state, machine learning is performed by the model training unit 4 to learn a correspondence relation between the explanatory variables and the target variables, that is, an input-output relation of the model 2.

In the present embodiment, the model 2 is a multi-layer neural network including an input layer, multiple intermediate layers, and an output layer, as illustrated in FIG. 1. The number of neurons provided in the input layer of the model 2 is the same as the number of items of explanatory variables, and values of respective items are input. In FIG. 1, the temperature T (°C) as the manufacturing condition and percentages by weight (wt%) of the three additive elements Si, Cu, and Mg as a material composition are respectively input to the neurons in the input layer. The number of neurons provided in the output layer of the model 2 is the same as the number of items of the target variables, and values of respective items are output.

For the output layer of the multi-layer neural network of the model 2, a softmax function is used. That is, each of the multiple outputs of the model 2 is in a range from 0 to 1, and the total sum of the multiple outputs is 1. As described above, in the present embodiment, the output of model 2 is the phase fraction of each compound in the thermodynamic equilibrium state. Therefore, by using the softmax function in the output layer, the output value of the model 2 can be used as the phase fraction with no additional operation, thereby reducing the calculation cost.

The training data generating unit 3 generates training data of the model 2. The training data includes inputs related to predetermined design conditions and outputs related to the thermodynamic equilibrium state that may occur based on the design conditions. The training data generating unit 3 generates explanatory variables including a combination of predetermined ranges of the design conditions (i.e., a composition and a manufacturing condition) of the aluminum alloy, calculates target variables by using a CALPHAD method, and generates training data including the generated explanatory variables and the calculated target variables.

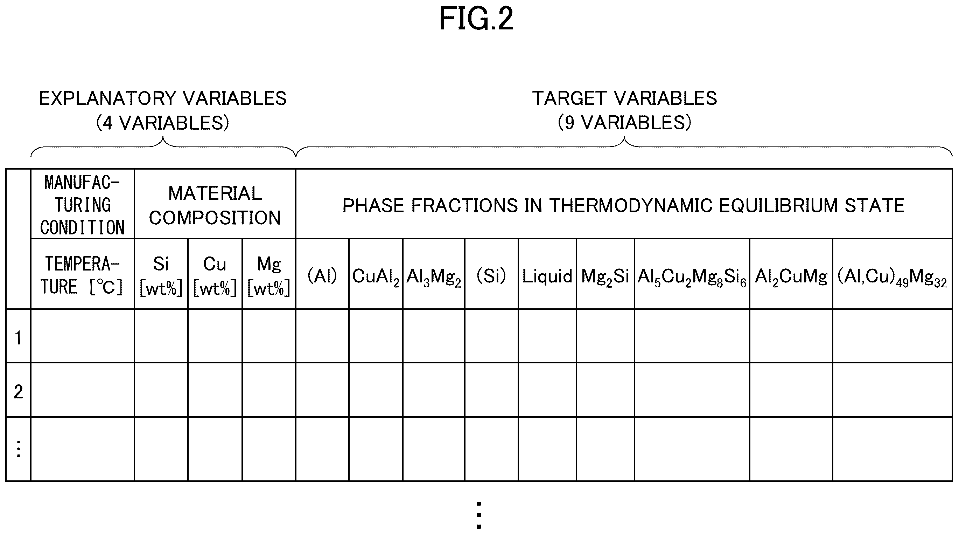

FIG. 2 is a drawing illustrating an example of training data of a four-component system. The training data in FIG. 2 is four-component system training data including, as explanatory variables, four items of the percentages by weight (wt%) of three additive elements Si, Cu, and Mg with respect to composition of the aluminum alloy and the temperature (°C) among manufacturing conditions, and as target variables, the phase fractions of nine compounds based on the input composition of three elements. If the four-component system training data is used, as illustrated in FIG. 1, the number of the neurons in the input layer in the model 2 is four and the number of the neurons in the output layer is nine.

The percentages by weight of the elements Si, Cu, and Mg, which are explanatory variables, are, for example, all combinations of value groups of the respective elements. For example, the value groups of the respective elements are generated by selecting values of the respective elements within predetermined ranges. Values of the temperature of the explanatory variable are a group of values selected within a predetermined temperature range (e.g., 0 to 1000°C). Each of all combinations of these compositions is combined with each of the group of values of the temperature to generate a set of explanatory variables. The number of compounds in the target variables is determined in accordance with contents of the compositions included in the explanatory variables.

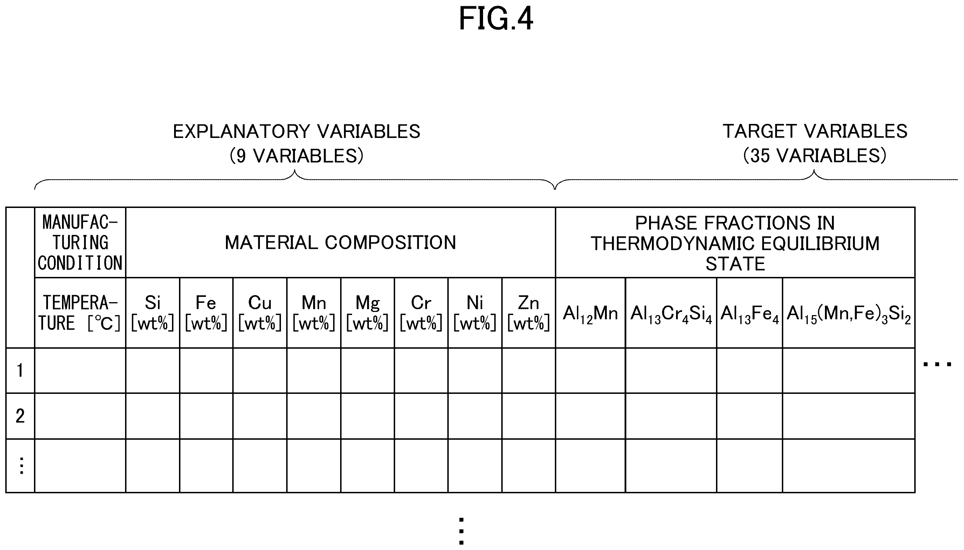

FIG. 4 is a drawing illustrating an example of training data of a nine-component system. The training data in FIG. 4 is nine-component system training data including, as explanatory variables, nine items of percentages by weight of eight additive elements Si, Fe, Cu, Mn, Mg, Cr, Ni, and Zn with respect to composition of the aluminum alloy, and the temperature (°C) of the manufacturing condition, and, as the target variables, phase fractions of 35 compounds based on the input composition of eight elements. If the nine-component system training data is used, the number of the neurons in the input layer of the model 2 would be 9 and the number of the neurons in the output layer would be 35.

Here, kinds of compositions to be included in the explanatory variables are not limited to the above described three kinds of the additive elements, eight kinds of the additive elements, and the like, and any kind and any number can be set. Additionally, items of the manufacturing conditions to be included in the explanatory variables may be conditions other than the temperature, and the gas atmosphere may be included.

The model training unit 4 performs machine learning so that the input-output relation of the model 2 approaches the input-output relation of the training data by using the training data generated by the training data generating unit 3. In the present embodiment, the model training unit 4 performs machine learning on the model 2 by using deep learning.

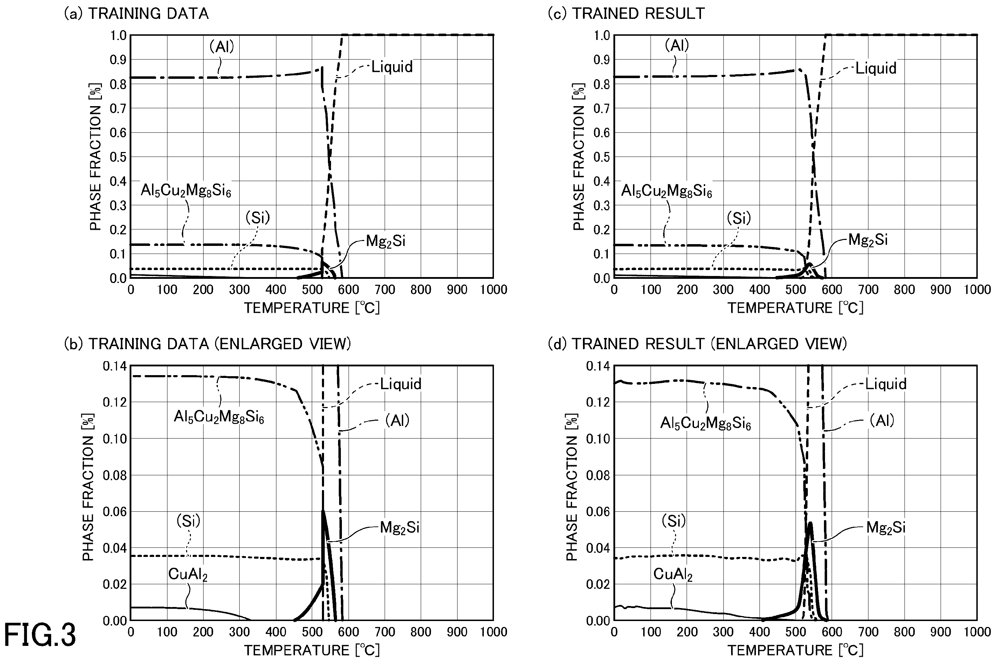

FIG. 3 is a drawing illustrating a trained result obtained when the four-component system training data illustrated in FIG. 2 is used. FIG. 3 (a) illustrates a phase diagram generated from the target variables of the training data, and FIG. 3 (b) illustrates an enlarged view of the phase diagram. FIG. 3 (c) illustrates a phase diagram generated from the outputs of the trained model 2 based on the input explanatory variables of the training data, and FIG. 3 (d) is an enlarged view of the phase diagram. The phase diagram is generated for each combination of percentages by weight of Si, Cu, and Mg in the target variables over the range of the temperature of the target variable. In the example of FIG. 3, the target variables are actually nine items, but, due to space limitation, representatives are illustrated. The horizontal axis of each phase diagram illustrated in FIG. 3 indicates the temperature (°C) and the vertical axis indicates a phase fraction of each compound in the thermodynamic equilibrium state. By comparing (a) and (b) in FIG. 3 with (c) and (d) in FIG. 3, it can be found that the output of the trained model 2 is close to the training data in the four-component system.

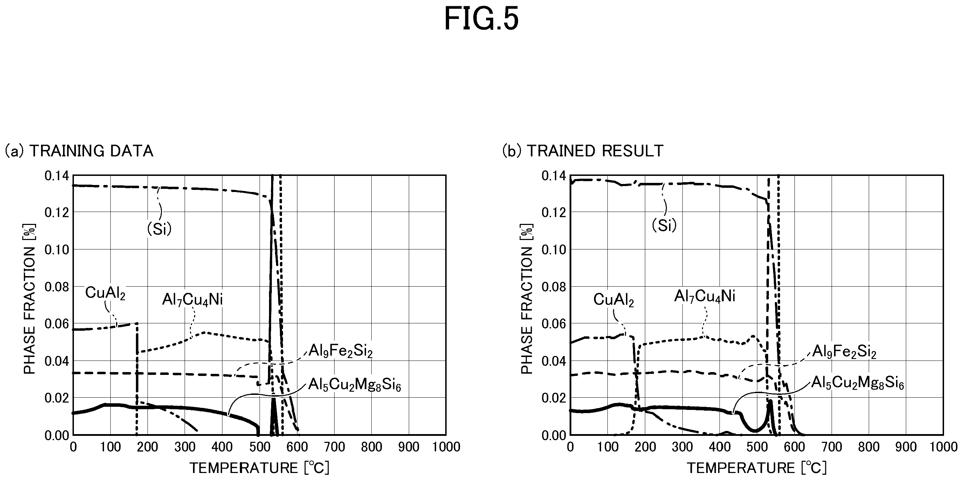

FIG. 5 is a drawing illustrating a trained result obtained when the nine-component system training data illustrated in FIG. 4 is used, FIG. 5 (a) illustrates a phase diagram generated from the target variables of the training data, and FIG. 5 (b) illustrates a phase diagram generated from the outputs of the trained model 2 based on the input explanatory variables of the training data. In the example of FIG. 5, the target variables are actually 35 items, but, due to space limitation, representatives are illustrated. By comparing FIG. 5 (a) with FIG. 5 (b), it can be found that the outputs of the trained model 2 are also close to the training data in the nine-component system, as in the four-component system.

The machine learning performed by the model training unit 4 on the model 2 may be configured to be performed so as to reduce the output error of each data set of the training data, or may be configured to be performed so that the phase diagram based on the outputs of the model 2 illustrated in FIG. 3 (c) or FIG. 5 (b) approaches the phase diagram of the training data illustrated in FIG. 3(a) or FIG. 5(a).

The explanatory variable setting unit 5 sets predictive explanatory variables that are used to predict the thermodynamic equilibrium state of the target material. For example, the explanatory variable setting unit 5 can set the explanatory variables by displaying an input screen of various design conditions on a GUI of a display device and prompting a designer to input the explanatory variables.

The prediction unit 6 outputs predictive target variables, which are predicted results of the thermodynamic equilibrium state, from the model 2, by inputting the predictive explanatory variables, set by the explanatory variable setting unit 5, into the model 2 on which the machine learning has been performed by the model training unit 4.

The phase diagram display unit 7 generates and displays a phase diagram of the thermodynamic equilibrium state of the target material based on the predictive target variables output from the prediction unit 6.

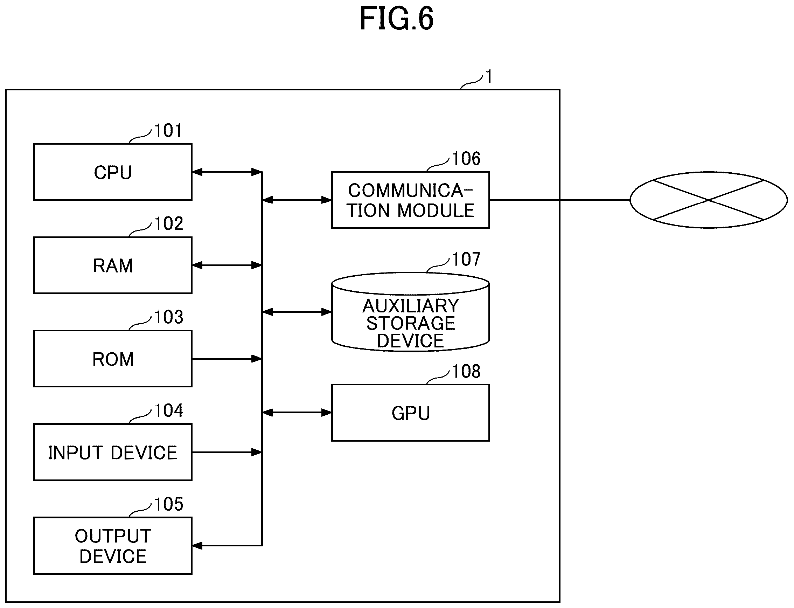

FIG. 6 is a block diagram illustrating the hardware configuration of the prediction device 1. As illustrated in FIG. 6, the prediction device 1 may be configured as a computer system that physically includes a central processing unit (CPU) 101, a graphics processing unit (GPU) 108, a random access memory (RAM) 102 and a read only memory (ROM) 103 that are main storage devices, an input device 104, such as a keyboard and a mouse, an output device 105 such as a display, a communication module 106 that is a data transmission and reception device such as a network card, an auxiliary storage device 107 such as a hard disk drive, and the like.

Each function of the prediction device 1 illustrated in FIG. 1 is achieved by reading predetermined computer software (i.e., a prediction program) on hardware such as the CPU 101 and the RAM 102, operating the communication module 106, the input device 104, and the output device 105 under control of the CPU 101, and reading and writing data in the RAM 102 and the auxiliary storage device 107. That is, by executing the prediction program according to the present embodiment on a computer, the prediction device 1 functions as the model 2, the training data generating unit 3, the model training unit 4, the explanatory variable setting unit 5, the prediction unit 6, and the phase diagram display unit 7 illustrated in FIG. 1.

The prediction program of the present embodiment is stored, for example, in a storage device provided in the computer. The prediction program may be configured such that a portion or an entirety of the prediction program is transmitted through a transmission medium such as a communication line and received and recorded (including installed) by the communication module 106 or the like provided in the computer. The prediction program may be configured to be recorded (including installed) in the computer from a state in which a portion or an entirety of the prediction program is stored in a portable storage medium such as a CD-ROM, a DVD-ROM, or a flash memory.

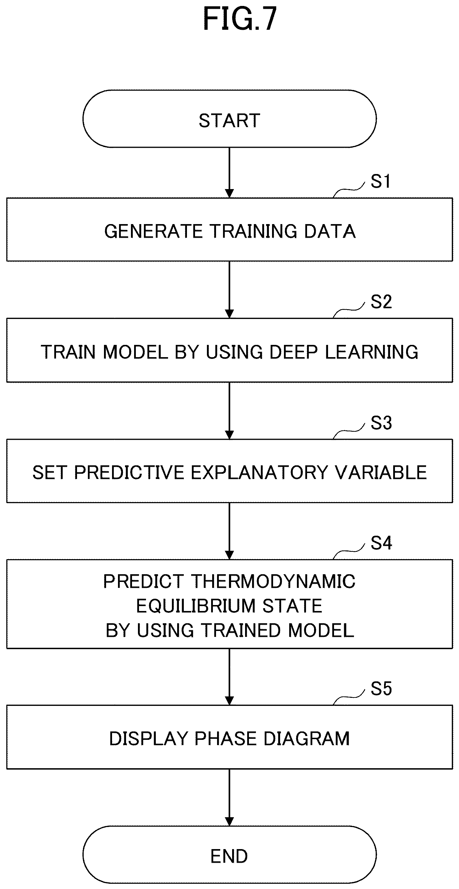

FIG. 7 is a flowchart of a prediction process of predicting the thermodynamic equilibrium state, performed by the prediction device 1 according to the embodiment.

In step S1, the training data for the model 2 is generated by the training data generating unit 3 (i.e., a training data generating step). The training data generating unit 3 generates a set of explanatory variables so as to, in accordance with an input of ranges of the compositions and the manufacturing conditions (i.e., temperature and the like) that are specified by a designer, cover the ranges, calculate the target variables by using a CALPHAD method using the set of all the generated explanatory variables, and generate, for example, the training data of the four-component system and the nine-component system illustrated in FIG. 2 and FIG. 4 by linking the target variables with the explanatory variables.

In step S2, the model training unit 4 performs the machine learning on the model 2 by using the training data generated in step S1 (i.e., a model training step). The model training unit 4 trains the model by adjusting weights between layers of a multi-layer neural network so that, in response to the explanatory variables of the training data being input, outputs match the target variables linked with the explanatory variables of the learning data. The model training unit 4 trains the model 2 by using, for example, deep learning.

In step S3, the explanatory variable setting unit 5 sets predictive explanatory variables used for predicting the thermodynamic equilibrium state of the target material (i.e., an explanatory variable setting step).

In step S4, the prediction unit 6 predicts the thermodynamic equilibrium state of the target material by using the trained model 2 (i.e., a prediction step). The prediction unit 6 inputs the explanatory variables set in step S3 into the model 2 on which the machine learning has been performed in step S2, and acquires predictive target variables that are predicted results of the thermodynamic equilibrium state output from the model 2.

In step S5, the phase diagram display unit 7 generates and displays a phase diagram of the thermodynamic equilibrium state of the target material based on the predictive target variables output from the prediction unit 6.

The effect of the present embodiment will be described. The prediction device of the present embodiment includes the model 2 that outputs target variables related to the thermodynamic equilibrium state based on input explanatory variables related to design conditions of a target material, a training data generating unit 3 that generates training data including inputs related to predetermined design conditions and outputs related to a thermodynamic equilibrium state that may occur based on the design conditions, a model training unit 4 that performs machine learning by using the training data generated by the training data generating unit 3 so that an input-output relation of the model 2 approaches an input-output relation of the training data, an explanatory variable setting unit 5 that sets predictive explanatory variables that are used to predict the thermodynamic equilibrium state of the target material, and a prediction unit 6 that outputs, from the model 2, predictive target variables that are predicted results of the thermodynamic equilibrium state based on the input predictive explanatory variables that are input to the model 2 on which the mechanical learning is performed by the model training unit 4.

With this configuration, by simply inputting the explanatory variables into the trained model 2, the predictive target variables that are the predicted results of the thermodynamic equilibrium state corresponding to the explanatory variables are output from the model 2, so that the cost of calculating the target variables is greatly reduced in comparison with a conventional CALPHAD simulation, thereby calculating the thermodynamic equilibrium state in a shorter period of time. For example, if a phase diagram of all compositions is calculated to screen 10,000 sets of explanatory variables (i.e., sets of compositions and manufacturing conditions), in a simulation, it takes about 90 seconds to calculate for each set, so about 250 hours would be required in total. However, in the embodiment of the present invention, it takes about 3 milliseconds to calculate for each set, so about 30 seconds are required in total.

With this configuration, appropriate predictive target variables with respect to unknown inputs that are different from the explanatory variables of the training data can be output by using a generalization capability of the trained model 2, thereby accurately predicting the thermodynamic equilibrium state. That is, in a conventional simulation method, a simulation operation is required to be performed again in a case of unknown inputs, but, in the present embodiment, if the trained model 2 is acquired, appropriate outputs can be obtained with respect to unknown inputs without performing additional training of the model 2. This enables comprehensive analysis of compositions to be performed in a short period of time and with high accuracy, so that a large amount of compositions that would be unrealistic to screen using conventional simulations can be screened, thereby extracting more optimum design conditions.

The prediction device 1 according to the present embodiment includes the phase diagram display unit 7 that generates and displays a phase diagram in the thermodynamic equilibrium state based on the predictive target variables that are output from the prediction unit 6. With this configuration, predicted results of the thermodynamic equilibrium state of the target material can be visually presented to a designer, so that the designer can easily understand the predicted results.

In the prediction device 1 according to the present embodiment, the model 2 is a multi-layer neural network, and the model training unit 4 trains the model using deep learning. Therefore, training can be performed at a high speed and with high accuracy, and the thermodynamic equilibrium state can be predicted with greater accuracy.

In the prediction device 1 according to the present embodiment, the target variables that are the outputs of the model 2 are phase fractions of the target material in the thermodynamic equilibrium state, and neurons in an output layer of the multi-layer neural network of the model 2 calculate output values based on a softmax function. With this configuration, the sum of the outputs can be always maintained at 1, so that, in the embodiment in which the phase fractions are the outputs of the model 2, each output can be used as the phase fraction without change, thereby further reducing the calculation cost.

Further, in the prediction device 1 according to the present embodiment, the training data generating unit 3 generates explanatory variables and calculates target variables by using a CALPHAD method to generate the training data. Therefore, the target variables can be accurately calculated, and the accuracy of the training data can be improved.

As described above, the present embodiment has been described with reference to the specific examples. However, the present disclosure is not limited to these specific examples. Examples, to which design modifications have been appropriately made by a person having an ordinary skill in the art, are also included in the present disclosure as long as the feature of the present disclosure is provided. The elements provided in each of the embodiments described above, and the arrangement, conditions, shape, and the like thereof are not limited to the examples, and may be appropriately modified. The combination of the elements provided by each of the above-described specific examples may be appropriately modified, as long as a technical inconsistency does not occur.

In the above-described embodiment, although an aluminum alloy is the target material of which the thermodynamic equilibrium state is predicted by the prediction device 1, an alloy system other than an aluminum alloy may be used as the target material. Examples of such alloy systems include Fe alloys, Cu alloys, Ni alloys, Co alloys, Ti alloys, Mg alloys, Mn alloys, Zn alloys, and the like. In addition to alloys, ceramics, aqueous solutions, chemical reactions, or the like may be used as the target material.

In the above-described embodiment, a multi-layer neural network is exemplified as the model 2, and deep learning is exemplified as a machine learning technique of the model 2. However, the model 2 and the learning technique are not limited thereto, and other supervised learning models, such as genetic algorithms, and other machine learning techniques, such as random forest regression and kernel ridge regression, can be used.

In the above-described embodiment, a configuration in which the training data generating unit 3 calculates and generates the training data for the model 2 by using a CALPHAD method is exemplified. However, a configuration, in which the training data is generated by using experimental results instead of simulation results in which the thermodynamic calculation or the like is performed, may be used. For example, a method, in which various alloy materials manufactured by changing compositions are maintained at various temperatures to reach equilibrium states, and then rapidly cooled to a low temperature to freeze the equilibrium states, to determine types and phase fractions of compounds in the various alloy materials by using various analyses, may be considered. As methods of the analyses, for example, a method of determining types of compounds from peak positions acquired by X-ray diffraction measurement and calculating phase fractions from the peak intensity ratio, and a method of performing energy dispersive X-ray spectroscopy (EDS) on an observed object field in electron microscopic observation, such as scanning electron microscope (SEM) and transmission electron microscope (TEM), to determine types of phases and calculate phase fractions from total area ratios of particles of respective phases in the observed image, may be considered.

In the above-described embodiment, a configuration, in which the prediction device 1 ultimately outputs a phase diagram of the thermodynamic equilibrium state at the phase diagram display unit 7, is exemplified. However, a configuration, in which the prediction device 1 outputs numerical values of predicted results output from the prediction unit 6 and does not output a phase diagram, may be used.

In the above embodiment, a configuration, in which the outputs of the model 2 are phase fractions of the compounds in the thermodynamic equilibrium state, is exemplified, but the outputs of the model 2 may be other than phase fractions as long as the outputs of the model 2 are information related to the thermodynamic equilibrium state.

The present international application is based on and claims priority to Japanese Patent Application No. 2018-206017, filed on October 31, 2018.

### Description of the Reference Numerals

1 prediction device for thermodynamic equilibrium state
2 model
3 training data generating unit
4 model training unit
5 explanatory variable setting unit
6 prediction unit
7 phase diagram display unit

## Claims

1. A prediction device for predicting a thermodynamic equilibrium state of a target material, comprising:
a model configured to output target variables related to the thermodynamic equilibrium state based on input explanatory variables related to design conditions of the target material;
a training data generating unit configured to generate training data including inputs related to predetermined design conditions and outputs related to the thermodynamic equilibrium state that may occur based on the predetermined design conditions;
a model training unit configured to perform machine learning by using the training data generated by the training data generating unit so that an input-output relation of the model approaches an input-output relation of the training data;
an explanatory variable setting unit configured to set predictive explanatory variables that are used to predict the thermodynamic equilibrium state of the target material;
a prediction unit configured to output predictive target variables from the model, based on the predictive explanatory variables, wherein the predictive explanatory variables are input into the model on which the machine learning has been performed by the model training unit, and wherein the predictive target variables are predicted results of the thermodynamic equilibrium state, and a design condition used to manufacture the target material is determined based on the predicted results of the thermodynamic equilibrium state; and
a phase diagram display unit configured to generate and display a phase diagram of the thermodynamic equilibrium state based on the predictive target variables output from the prediction unit.

2. The prediction device as claimed in claim 1, wherein the model is a multi-layer neural network and the model training unit trains the model by using deep learning.

3. The prediction device as claimed in claim 2,
wherein the target variables that are output from the model are phase fractions of the target material in the thermodynamic equilibrium state; and
wherein a softmax function is used for neurons in an output layer of the multi-layer neural network.

4. The prediction device as claimed in any one of claims 1 to 3, wherein the training data generating unit generates the explanatory variables including a combination of predetermined ranges of the design conditions, calculates the target variables by using a CALPHAD method, and generates the training data including the generated explanatory variables and the calculated target variables.

5. The prediction device as claimed in any one of claims 1 to 4, wherein the target material is an aluminum alloy, the explanatory variables include a composition and a manufacturing condition of the aluminum alloy, and the target variables include phase fractions of the aluminum alloy in the thermodynamic equilibrium state.

6. A prediction method for predicting a thermodynamic equilibrium state of a target material, wherein the prediction method is carried out by a computer and comprises:
a training data generating step of generating training data including inputs related to predetermined design conditions and outputs related to the thermodynamic equilibrium state that may occur based on the predetermined design conditions, for a model configured to output target variables related to the thermodynamic equilibrium state based on input explanatory variables related to design conditions of the target material;
a model training step of performing machine learning by using the training data generated by the training data generating step so that an input-output relation of the model approaches an input-output relation of the training data;
an explanatory variable setting step of setting predictive explanatory variables that are used to predict the thermodynamic equilibrium state of the target material;
a prediction step of outputting predictive target variables from the model, based on the predictive explanatory variables, wherein the predictive explanatory variables are input into the model on which the machine learning has been performed by the model training step, and wherein the predictive target variables are predicted results of the thermodynamic equilibrium state, and a design condition used to manufacture the target material is determined based on the predicted results of the thermodynamic equilibrium state; and
a phase diagram display step of generating and displaying a phase diagram of the thermodynamic equilibrium state based on the predictive target variables output from the prediction step.

7. A prediction program for predicting a thermodynamic equilibrium state of a target material, the prediction program causing a computer to achieve functions comprising:
a training data generating function of generating training data including inputs related to predetermined design conditions and outputs related to the thermodynamic equilibrium state that may occur based on the predetermined design conditions, for a model configured to output target variables related to the thermodynamic equilibrium state based on input explanatory variables related to design conditions of the target material;
a model training function of performing machine learning by using the training data generated by the training data generating function so that an input-output relation of the model approaches an input-output relation of the training data;
an explanatory variable setting function of setting predictive explanatory variables that are used to predict the thermodynamic equilibrium state of the target material;
a prediction function of outputting predictive target variables from the model, based on the predictive explanatory variables, wherein the predictive explanatory variables are input into the model on which the machine learning has been performed by the model training function, and wherein the predictive target variables are predicted results of the thermodynamic equilibrium state, and a design condition used to manufacture the target material is determined based on the predicted results of the thermodynamic equilibrium state; and
a phase diagram display function of generating and displaying a phase diagram of the thermodynamic equilibrium state based on the predictive target variables output from the prediction function.

## Patentansprüche

1. Vorhersagevorrichtung zur Vorhersage eines thermodynamischen Gleichgewichtszustands eines Zielmaterials, umfassend:
ein Modell, das ausgelegt ist, um Zielvariablen betreffend den thermodynamischen Gleichgewichtszustand basierend auf erläuternden Eingabevariablen betreffend Aufbaubedingungen des Zielmaterials auszugeben;
eine Trainingsdatenerzeugungseinheit, die ausgelegt ist, um Trainingsdaten, einschließlich Eingaben betreffend vorbestimmten Aufbaubedingungen und Ausgaben betreffend den thermodynamischen Gleichgewichtszustand, der basierend auf den vorbestimmten Aufbaubedingungen eintreten kann, zu erzeugen;
eine Modelltrainingseinheit, die ausgelegt ist, um maschinelles Lernen unter Verwendung der Trainingsdaten, die durch die Trainingsdatenerzeugungseinheit erzeugt wurden, durchzuführen, sodass sich eine Eingabe-Ausgabe-Beziehung des Modells an eine Eingabe-Ausgabe-Beziehung der Trainingsdaten annähert;
eine Einstelleinheit für die erläuternde Variable, die ausgelegt ist, um prädiktive erläuternde Variablen einzustellen, die verwendet werden, um den thermodynamischen Gleichgewichtszustand des Zielmaterials vorherzusagen;
eine Vorhersageeinheit, die ausgelegt ist, um prädiktive erläuternde Variablen aus dem Modell basierend auf den prädiktiven erläuternden Variablen auszugeben, wobei die prädiktiven erläuternden Variablen in das Modell, auf dem das maschinelle Lernen von der Modelltrainingseinheit durchgeführt wurde, eingegeben werden und wobei die prädiktiven Zielvariablen vorhergesagte Ergebnisse des thermodynamischen Gleichgewichtszustands sind und eine Aufbaubedingung, die verwendet wird, um das Zielmaterial herzustellen, basierend auf den vorhergesagten Ergebnissen des thermodynamischen Gleichgewichtszustands bestimmt wird; und
eine Phasendiagramm-Anzeigeeinheit, die ausgelegt ist, um ein Phasendiagramm des thermodynamischen Gleichgewichtszustands basierend auf den prädiktiven Zielvariablen, die aus der Vorhersageeinheit ausgegeben wurden, zu erzeugen und zu zeigen.

2. Vorhersagevorrichtung nach Anspruch 1, wobei das Modell ein mehrschichtiges neuronales Netz ist und die Modelltrainingseinheit das Modell unter Verwendung von Deep Learning trainiert.

3. Vorhersagevorrichtung nach Anspruch 2, wobei die Zielvariablen, die aus dem Modell ausgegeben werden, Phasenfraktionen des Zielmaterials in dem thermodynamischen Gleichgewichtszustand sind; und
wobei eine Softmax-Funktion für Neuronen in einer Ausgabeschicht des mehrschichtigen neuronalen Netzes verwendet wird.

4. Vorhersagevorrichtung nach einem der Ansprüche 1 bis 3, wobei die Trainingsdatenerzeugungseinheit die erläuternden Variablen, einschließlich einer Kombination von vorbestimmten Bereichen der Aufbaubedingungen, erzeugt, die Zielvariablen unter Verwendung eines CALPHAD-Verfahrens berechnet und die Trainingsdaten, einschließlich der erzeugten erläuternden Variablen und der berechneten Zielvariablen, erzeugt.

5. Vorhersagevorrichtung nach einem der Ansprüche 1 bis 4, wobei das Zielmaterial eine Aluminiumlegierung ist, wobei die erläuternden Variablen eine Zusammensetzung und eine Herstellungsbedingung der Aluminiumlegierung umfassen und die Zielvariablen Phasenfraktionen der Aluminiumlegierung in dem thermodynamischen Gleichgewichtszustand umfassen.

6. Vorhersageverfahren zum Vorhersagen eines thermodynamischen Gleichgewichtszustands eines Zielmaterials, wobei das Vorhersageverfahren durch einen Computer ausgeführt wird und Folgendes umfasst:
einen Trainingsdaten-Erzeugungsschritt des Erzeugens von Trainingsdaten, einschließlich Eingaben betreffend vorbestimmten Aufbaubedingungen und Ausgaben betreffend den thermodynamischen Gleichgewichtszustand, der basierend auf den vorbestimmten Aufbaubedingungen eintreten kann, für ein Modell, das ausgelegt ist, um Zielvariablen betreffend den thermodynamischen Gleichgewichtszustand basierend auf erläuternden Eingabevariablen betreffend Aufbaubedingungen des Zielmaterials auszugeben;
einen Modelltrainingsschritt des Durchführens von maschinellem Lernen unter Verwendung der Trainingsdaten, die durch den Trainingsdaten-Erzeugungsschritt erzeugt wurden, sodass sich eine Eingabe-Ausgabe-Beziehung des Modells an eine Eingabe-Ausgabe-Beziehung der Trainingsdaten annähert;
einen Einstellschritt einer erläuternden Variable des Einstellens von prädiktiven erläuternden Variablen, die verwendet werden, um den thermodynamischen Gleichgewichtszustand des Zielmaterials vorherzusagen;
einen Vorhersageschritt des Ausgebens von prädiktiven Zielvariablen aus dem Modell basierend auf den prädiktiven erläuternden Variablen, wobei die prädiktiven erläuternden Variablen in das Modell, auf dem das maschinelle Lernen durch den Modelltrainingsschritt durchgeführt wurde, eingegeben werden und wobei die prädiktiven Zielvariablen vorhergesagte Ergebnisse des thermodynamischen Gleichgewichtszustands sind und eine Aufbaubedingung, die verwendet wird, um das Zielmaterial herzustellen, basierend auf den vorhergesagten Ergebnissen des thermodynamischen Gleichgewichtszustands bestimmt wird; und
einen Phasendiagrammanzeigeschritt des Erzeugens und Anzeigens eines Phasendiagramms des thermodynamischen Gleichgewichtszustands basierend auf den prädiktiven Zielvariablen, die aus dem Vorhersageschritt ausgegeben wurden.

7. Vorhersageprogramm zum Vorhersagen eines thermodynamischen Gleichgewichtszustands eines Zielmaterials, wobei das Vorhersageprogramm bewirkt, dass ein Computer Funktionen erzielt, die Folgendes umfassen:
eine Trainingsdatenerzeugungsfunktion des Erzeugens von Trainingsdaten, einschließlich Eingaben betreffend vorbestimmten Aufbaubedingungen und Ausgaben betreffend den thermodynamischen Gleichgewichtszustand, der basierend auf den vorbestimmten Aufbaubedingungen eintreten kann, für ein Modell, das ausgelegt ist, um Zielvariablen betreffend den thermodynamischen Gleichgewichtszustand basierend auf erläuternden Eingabevariablen betreffend Aufbaubedingungen des Zielmaterials auszugeben;
eine Modelltrainingsfunktion des Durchführens von maschinellem Lernen unter Verwendung der Trainingsdaten, die durch die Trainingsdatenerzeugungsfunktion erzeugt wurden, sodass sich eine Eingabe-Ausgabe-Beziehung des Modells an eine Eingabe-Ausgabe-Beziehung der Trainingsdaten annähert;
eine Einstellfunktion einer erläuternden Variable des Einstellens von prädiktiven erläuternden Variablen, die verwendet werden, um den thermodynamischen Gleichgewichtszustand des Zielmaterials vorherzusagen;
eine Vorhersagefunktion des Ausgebens von prädiktiven Zielvariablen aus dem Modell basierend auf den prädiktiven erläuternden Variablen, wobei die prädiktiven erläuternden Variablen in das Modell, auf dem das maschinelle Lernen durch die Modelltrainingsfunktion durchgeführt wurde, eingegeben werden und wobei die prädiktiven Zielvariablen vorhergesagte Ergebnisse des thermodynamischen Gleichgewichtszustands sind und eine Aufbaubedingung, die verwendet wird, um das Zielmaterial herzustellen, basierend auf den vorhergesagten Ergebnissen des thermodynamischen Gleichgewichtszustands bestimmt wird; und
eine Phasendiagrammanzeigefunktion des Erzeugens und Anzeigens eines Phasendiagramms des thermodynamischen Gleichgewichtszustands basierend auf den prädiktiven Zielvariablen, die aus der Vorhersagefunktion ausgegeben wurden.

## Revendications

1. Dispositif de prédiction destiné à prédire un état d'équilibre thermodynamique d'un matériau cible, comprenant :
un modèle configuré pour fournir en sortie des variables cibles liées à l'état d'équilibre thermodynamique sur la base de variables explicatives d'entrée liées à des conditions de conception du matériau cible ;
une unité de génération de données d'entraînement configurée pour générer des données d'entraînement comprenant des entrées liées à des conditions de conception prédéterminées et des sorties liées à l'état d'équilibre thermodynamique qui peut se produire sur la base des conditions de conception prédéterminées ;
une unité d'entraînement de modèle configurée pour effectuer un apprentissage automatique en utilisant les données d'entraînement générées par l'unité de génération de données d'entraînement de sorte qu'une relation entrée-sortie du modèle se rapproche d'une relation entrée-sortie des données d'entraînement ;
une unité de définition de variables explicatives configurée pour définir des variables explicatives prédictives qui sont utilisées pour prédire l'état d'équilibre thermodynamique du matériau cible ;
une unité de prédiction configurée pour fournir en sortie des variables cibles prédictives à partir du modèle, sur la base des variables explicatives prédictives, dans lesquelles les variables explicatives prédictives sont introduites dans le modèle sur lequel l'apprentissage automatique a été effectué par l'unité d'entraînement de modèle, et dans lesquelles les variables cibles prédictives sont des résultats prédits de l'état d'équilibre thermodynamique, et une condition de conception utilisée pour fabriquer le matériau cible est déterminée sur la base des résultats prédits de l'état d'équilibre thermodynamique ; et
une unité d'affichage de diagramme de phase configurée pour générer et afficher un diagramme de phase de l'état d'équilibre thermodynamique sur la base des variables cibles prédictives fournies en sortie à partir de l'unité de prédiction.

2. Dispositif de prédiction selon la revendication 1, dans lequel le modèle est un réseau de neurones multicouche et l'unité d'entraînement de modèle entraîne le modèle en utilisant l'apprentissage profond.

3. Dispositif de prédiction selon la revendication 2,
dans lequel les variables cibles qui sont fournies en sortie par le modèle sont des fractions de phase du matériau cible dans l'état d'équilibre thermodynamique ; et
dans lequel une fonction « softmax » est utilisée pour les neurones dans une couche de sortie du réseau de neurones multicouche.

4. Dispositif de prédiction selon l'une quelconque des revendications 1 à 3, dans lequel l'unité de génération de données d'entraînement génère les variables explicatives en incluant une combinaison de plages prédéterminées des conditions de conception, calcule les variables cibles en utilisant une méthode CALPHAD, et génère les données d'entraînement comprenant les variables explicatives générées et les variables cibles calculées.

5. Dispositif de prédiction selon l'une quelconque des revendications 1 à 4, dans lequel le matériau cible est un alliage d'aluminium, les variables explicatives comprennent une composition et une condition de fabrication de l'alliage d'aluminium, et les variables cibles comprennent des fractions de phase de l'alliage d'aluminium dans l'état d'équilibre thermodynamique.

6. Procédé de prédiction pour prédire un état d'équilibre thermodynamique d'un matériau cible, dans lequel le procédé de prédiction est mis en œuvre par un ordinateur et comprend :
une étape de génération de données d'entraînement consistant à générer des données d'entraînement comprenant des entrées liées à des conditions de conception prédéterminées et des sorties liées à l'état d'équilibre thermodynamique qui peut se produire sur la base des conditions de conception prédéterminées, pour un modèle configuré pour fournir en sortie des variables cibles liées à l'état d'équilibre thermodynamique sur la base de variables explicatives d'entrée liées aux conditions de conception du matériau cible ;
une étape d'entraînement de modèle consistant à effectuer un apprentissage automatique en utilisant les données d'entraînement générées par l'étape de génération de données d'entraînement de sorte qu'une relation entrée-sortie du modèle se rapproche d'une relation entrée-sortie des données d'entraînement ;
une étape de définition de variables explicatives consistant à définir des variables explicatives prédictives qui sont utilisées pour prédire l'état d'équilibre thermodynamique du matériau cible ;
une étape de prédiction consistant à fournir en sortie des variables cibles prédictives à partir du modèle, sur la base des variables explicatives prédictives, dans lesquelles les variables explicatives prédictives sont introduites dans le modèle sur lequel l'apprentissage automatique a été effectué par l'étape d'entraînement de modèle, et dans lesquelles les variables cibles prédictives sont des résultats prédits de l'état d'équilibre thermodynamique, et une condition de conception utilisée pour fabriquer le matériau cible est déterminée sur la base des résultats prédits de l'état d'équilibre thermodynamique ; et
une étape d'affichage de diagramme de phase consistant à générer et à afficher un diagramme de phase de l'état d'équilibre thermodynamique sur la base des variables cibles prédictives fournies en sortie à partir de l'étape de prédiction.

7. Programme de prédiction destiné à prédire un état d'équilibre thermodynamique d'un matériau cible, le programme de prédiction amenant un ordinateur à réaliser des fonctions comprenant :
une fonction de génération de données d'entraînement consistant à générer des données d'entraînement comprenant des entrées liées à des conditions de conception prédéterminées et des sorties liées à l'état d'équilibre thermodynamique qui peut se produire sur la base des conditions de conception prédéterminées, pour un modèle configuré pour fournir en sortie des variables cibles liées à l'état d'équilibre thermodynamique sur la base de variables explicatives d'entrée liées à des conditions de conception du matériau cible ;
une fonction d'entraînement de modèle consistant à effectuer un apprentissage automatique en utilisant les données d'entraînement générées par la fonction de génération de données d'entraînement de sorte qu'une relation entrée-sortie du modèle se rapproche d'une relation entrée-sortie des données d'entraînement ;
une fonction de définition de variables explicatives consistant à définir des variables explicatives prédictives qui sont utilisées pour prédire l'état d'équilibre thermodynamique du matériau cible ;
une fonction de prédiction consistant à fournir en sortie des variables cibles prédictives à partir du modèle, sur la base des variables explicatives prédictives, dans lesquelles les variables explicatives prédictives sont introduites dans le modèle sur lequel l'apprentissage automatique a été effectué par la fonction d'entraînement de modèle, et dans lesquelles les variables cibles prédictives sont des résultats prédits de l'état d'équilibre thermodynamique, et une condition de conception utilisée pour fabriquer le matériau cible est déterminée sur la base des résultats prédits de l'état d'équilibre thermodynamique ; et
une fonction d'affichage de diagramme de phase consistant à générer et à afficher un diagramme de phase de l'état d'équilibre thermodynamique sur la base des variables cibles prédictives fournies en sortie à partir de la fonction de prédiction.
